# EUROPEAN PATENT SPECIFICATION

(11) **EP 2 613 746 B1**
(45) Date of publication and mention of the grant of the patent: **14.10.2015**
(21) Application number: 11755149.9
(22) Date of filing: 24.08.2011
(51) Int. Cl.: A61F 5/058

(54) **APPARATUS FOR TREATING CLAVICLE FRACTURES**
VORRICHTUNG ZUR BEHANDLUNG VON SCHLÜSSELBEINFRAKTUREN
APPAREIL POUR TRAITER DES FRACTURES DE LA CLAVICULE

(30) Priority: 28.12.2010 FR 1061325; 10.09.2010 FR 1057192
(43) Date of publication of application: 17.07.2013
(73) Proprietor: Whitechurch, Patrick, 69005 Lyon (FR)
(72) Inventor: Whitechurch, Patrick, 69005 Lyon (FR)
(74) Representative: Jeannet, Olivier
(86) International application number: PCT/IB2011/053712
(87) International publication number: WO 2012/032429

(56) References cited:
- WO-A1-2009/093180
- FR-A1- 2 880 797
- US-A- 1 585 341
- US-A- 3 277 889
- US-A- 3 499 441

## Description

The present invention relates to an apparatus for treating clavicle fractures.

Treating clavicle fracture involves keeping the patient's shoulders back during a minimum period of approximately two months in adults and one month in children. Keeping the shoulders back is essential to obtain the best possible alignment of the bone fragments in order to give the clavicle its normal length again.

To achieve this maintenance in position, it is currently known to use an apparatus commonly called "clavicle straps," comprising two straps joined together by one of their ends. This end is placed on the patient's back, then the straps are engaged on the shoulders, in the axillae, and brought back to the level of said end, to which they are attached and stretched.

This apparatus, notwithstanding the relatively significant width of the straps (about 60 mm), has the major drawback of causing a prolonged striction at the axillae, which can sometimes lead to phlebitis of one or both of the upper limbs and compression of the nerve trunks that can cause formication in the upper limbs, reaching the hands in some cases.

The publication of patent application N° US 3 499 441 describes an apparatus for treating clavicle fractures, comprising:
- a clothing able to surround the patient's shoulders and the upper part of the patient's arms and thorax in an adjusted manner, comprising cutouts in its areas intended to extend at the patient's axillae;
- elongate anterior reinforcements, connected to the clothing;
- a tightening means situated at the dorsal part of the clothing, able to perform tightening at the patient's shoulder blades.

This apparatus does not make it possible to resolve the aforementioned drawback.

Patent application publications N°WO2009/093180, FR2880797, DE 231 054 C, US 1 585 341 and US 3 277 889 illustrate various other apparatuses according to the prior art.

The present invention aims to resolve the aforementioned drawback, without lessening the quality of the treatment performed on the clavicle.

The concerned apparatus includes the aforementioned characteristics known from document no. US 3 499 441.

According to the invention,
- said elongate anterior reinforcements comprise two reinforcements intended to extend at the patient's delto-pectoral grooves when the apparatus is worn, and
- said tightening means is such that it can perform essentially transverse tightening, over at least the entire height of the shoulder blades.

The apparatus according to the invention thus does not comprise any straps passing at the patient's axillae, and on the contrary has cutout areas at said axillae. The patient's axillae are therefore practically not stressed by wearing the device. The shoulders are kept back through the combined actions of the adjusted clothing, two anterior reinforcements, which prevent the shoulders from sliding forward inside said clothing, and implementing essential dorsal transverse dorsal tightening, but also slightly downward, at the shoulder blades, over at least the height of the latter therefore over an extended height area.

The clothing thus assumes substantially the shape of a "bolero," able to cover the shoulders and the upper part of the patient's arms and thorax. In the height direction, this clothing extends substantially above the majority of the patient's pectorals, with an interruption on that side of the nipples. For women, the clothing can comprise an indentation making it possible to limit compression of the chest.

The clothing advantageously comprises an anterior middle part that is elastic over its entire height, said anterior middle part being able to be stretched in the transverse direction of the clothing, i.e. in a substantially horizontal direction when said clothing is worn.

The elastic anterior part increases the capacity of the clothing to allow the projection of the shoulders rearward during implementation of the dorsal tightening, and consequently allows a perfect positioning of the shoulders suitable for repairing a clavicle.

Said anterior middle part can be formed, by a single elastic band, or by a series of stepped elastic bands, for example three.

The clothing can comprise traditional sleeves, i.e., whereof the lower openings are delimited, in the circumferential direction, by continuous parts of the clothing, and are therefore not likely to be substantially enlarged or tightened; preferably, however, the clothing comprises sleeves having lateral slots that emerge in the lower openings of said sleeves, and a means for reclosing said lower openings.

The lower openings of the sleeves can thus be adapted to the different sizes of patients' arms.

Said slots can be situated at the rear part, the lateral part or even the forward part of the sleeves.

Over at least one lower sleeve opening, said reclosing means can in particular be formed by a pair of complementary self-gripping bands of the Velcro® type, one band of said pair being fastened on one of the parts of the sleeve delimiting said slot and the complementary band being fastened on the other of said parts and protruding therefrom so as to be able to cover the first band and to engage with it.

The clothing can also comprise forward openings favoring aeration.

The apparatus exists in several sizes, adapted to the patient's morphology, such that the clothing is adjusted to the patient.

The clothing can be made from any type of common non-allergenic fabric or tissue having an elastic flexibility, in particular from cotton, synthetic fibers or a mixture of the two. It can have a bias, possibly padded, at all of its edges.

The reinforcements must be longitudinally extendable, or slightly longitudinally extendable, and have a slight degree of elastic flexibility in the direction transverse to their length. They can be made from any common synthetic material, in particular silicone.

These reinforcements can be connected to the clothing in a fixed manner, using all suitable means, in particular sewing, adhesion, fusion of materials.

These reinforcements can also be removable relative to the clothing, and the apparatus can comprise series of reinforcements of different dimensions, such that the dimensions of the reinforcements can be adapted to the morphology of the patient to be treated. According to one possible embodiment of the invention in that case, the clothing includes, fastened thereto, one of the types of bands of a pair of complementary self-gripping bands of the Velcro® type, and each reinforcement comprises the other type of these bands, fastened to its surface intended to be pressed against the clothing.

Preferably, the removable connecting means of a reinforcement to the clothing is such that it allows the reinforcement to be connected in several locations on the clothing.

The position of this reinforcement relative to the clothing can thus be adjusted.

In particular, in this case, the first aforementioned type of band can have dimensions larger than those of a reinforcement.

According to another possible embodiment of the invention, the means for connecting a reinforcement to the clothing is made by a rebate that can be folded down on the reinforcement and be fastened to the clothing, in particular using complementary self-gripping strips of the Velcro® type.

Said essentially transverse tightening means preferably comprises several stepped fasteners situated at the shoulder blades, connected to the strap parts that are fastened to the clothing.

These stepped fasteners can thus be tightened independently of each other, which makes it possible to perform controlled tightening in different locations of the clothing, and to adapt this tightening to the patient's morphology.

The fasteners can be of any type, in particular with straps provided with complementary self-gripping strips, able to be passed into loops then reclosed on themselves stressed, or fastening parts that can be fastened to each other by clipping.

The tightening area extends substantially from the second thoracic vertebra to the seventh thoracic vertebra.

For proper understanding, the invention is described below in reference to the appended diagrammatic drawing, which shows, as non-limiting examples, several possible embodiments of the apparatus it concerns.
Figure 1 is a front view, when worn on a patient, according to a first embodiment; a reinforcement comprised by the apparatus is shown, on the right side of the figure, in the assembly position of clothing also comprised by said apparatus, and, on the left side of the figure, in the disassembled position;
figure 2 is a back view of the apparatus, when worn by a patient;
figure 3 is a cross-sectional view along line III-III of figure 1;
figure 4 is a cross-sectional view along line IV-IV of figure 1;
figure 5 is a cross-sectional view along line V-V of figure 2;
figure 6 is a view similar to figure 1, according to a second embodiment;
figure 7 is a cross-sectional view along line VII-VII of figure 6;
figure 8 is a view similar to figure 1, according to a third embodiment;
figure 9 is a view similar to figure 2, according to this third embodiment;
figure 10 is a view similar to figure 1, according to a fourth embodiment; and
figure 11 is a side view according to this fourth embodiment.

For simplification, the parts or elements that are identical or similar from one embodiment to the next will be designated using the same numerical references and will not be described again.

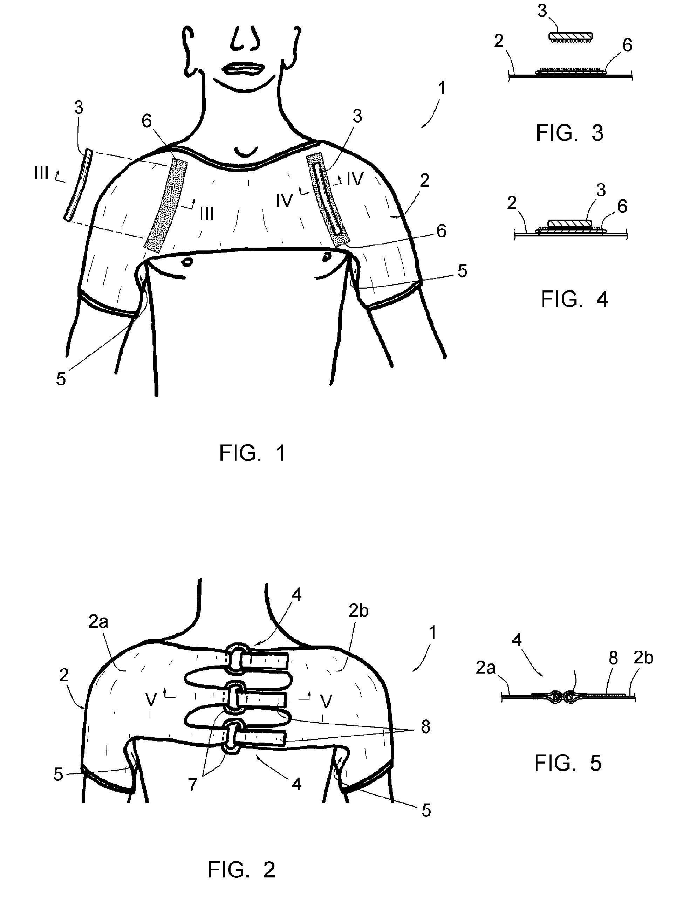

Figures 1 to 5 show an apparatus 1 for treating clavicle fractures, which comprises clothing 2, two elongate forward reinforcements 3 and three dorsal fasteners 4.

The clothing 2 substantially assumes the form of a "bolero," i.e. is able to surround the shoulders and the upper part of the patient's arms and thorax in an adjusted manner. In the height direction, on the chest side, it extends substantially above the majority of the patient's pectorals, with an interruption on that side of the nipples, and, on the back side, it extends substantially above the shoulder blades. On that same side of the back, the clothing is vertically separated into a left part 2a and a right part 2b, able to be tightened one towards the other using fasteners 4.

The clothing 2 can be made from any common type of non-allergenic fabric or cloth, having an elastic flexibility, in particular cotton, synthetic fibers or a mixture of the two.

It comprises cutouts 5 in its areas intended to extend at the patient's axillae, so that there is no tissue present in said axillae.

The clothing 2 also includes, on its forward side, at its areas extending above the delto-pectoral grooves of the patient when the apparatus 1 is worn, two strips 6 sewn to it, including one of the two types of materials comprised by a pair of complementary self-gripping strips of the Velcro® type, for example the hook material as shown in figure 3.

Each forward reinforcement 3 is formed by a blade made from silicone or another inextensible or slightly extensible material, and having a slight degree of elastic flexibility, so that the reinforcement 3 itself has said degree of elastic flexibility in a direction transverse to its length. On its surface intended to be pressed against the clothing 2, the reinforcement 3 comprises a pair of complementary self-gripping strips of the Velcro® type, for example the looped material as shown in figure 3. The reinforcement 3 can thus be connected removably to the clothing 2.

Each strip 6 has a width and length greater than those of the corresponding reinforcement 3, so that said reinforcement 3 can be positioned on the strip 6 of the clothing 2 in several locations of said clothing. An adjustment of the position of the reinforcement 3 relative to the clothing 2 is thus made possible.

The three dorsal fasteners 4 are situated at the shoulder blades, and are stepped consecutively to each other in the vertical direction, while being oriented so as to perform an essentially transverse tightening, but also slightly downward, at said shoulder blades, over the entire height thereof.

As shown in figures 2 and 5, each fastener 4 is formed by:
- two transverse extensions comprised by the left and right parts 2a, 2b of the clothing 2,
- an annular loop 7 connected to the transverse extension formed by the left part 2a, and
- a strip 8 extending the transverse extension formed by the right part.

The strip 8 can be engaged through the loop 7, then folded down against the transverse extension to which it is connected. The surface of said strip 8 situated opposite the transverse extension includes one of the two materials of a pair of complementary self-gripping materials of the Velcro® type while the surface of the transverse extension situated opposite the strip 8 includes the other of said two materials. After passing through the loop 7, each strip 8 is thus able to be connected to the corresponding transverse extension, while being immobilized relative thereto, with adjustment of the tension being done.

In practice, the clothing 2 adapted to the size, gender and morphology of the patient is chosen from among a series of clothing 2 of different sizes, such that said clothing 2 is adjusted to the patient's torso and shoulders, then the clothing is put on by the patient and the reinforcements 3 are placed on the strips 6, with an adjustment of their positions owing to the respective dimensions of said strips 6 and reinforcements 3.

The fasteners 4 are then stressed independently of each other, which makes it possible to perform essentially transverse tightening, but also oriented slightly downwards, which is checked at different locations of the clothing 2, and can be adapted to the patient's morphology.

It thus appears that the apparatus 1 does not comprise any straps at the patient's axillae, and on the contrary cutout areas 5 at said axillae. The patient's axillae are therefore practically not stressed by wearing the apparatus 1. The shoulders are kept back by the combined actions of the adjusted clothing 2, two front reinforcements 3, which prevent the shoulders from sliding forward inside said clothing 2, and the implementation of dorsal tightening using three fasteners 4, this tightening being done essentially transversely, but also slightly downwards, at the shoulder blades, on at least the height of the latter therefore over a height-extended area.

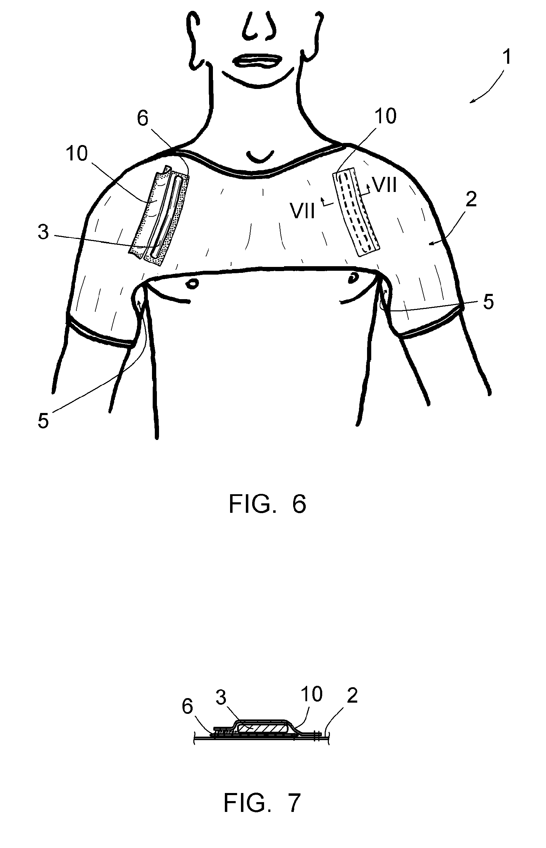

Figures 6 and 7 shows another possible embodiment of the apparatus 1, in which the means for connecting a reinforcement 3 to the clothing 2 are formed by a rebate 10 that can be folded down on the reinforcement 3 and fastened to the clothing 2, in particular using complementary self-gripping coverings of the Velcro® type.

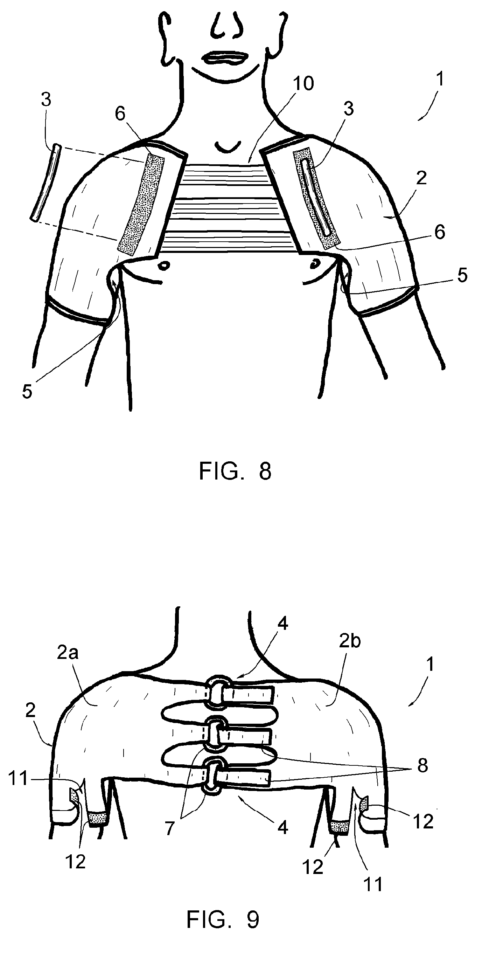

Figures 8 and 9 show another possible embodiment of the apparatus 1, in which the clothing 2 comprises, over the entire height of its forward side, a forward median part 10 that is elastic, formed by three stepped elastic strips. This forward median part 10 is able to be stretched in the transverse direction of the clothing 2, i.e. in a substantially horizontal direction when the clothing 2 is worn.

This elastic forward part 10 increases the capacity of the clothing 2 to allow the shoulders to be projected rearward upon implementation of the dorsal tightening, and consequently allows perfect positioning of the shoulders in a manner suitable for repairing a clavicle.

In this same embodiment, the clothing 2 comprises sleeves having, at their rear position, lateral slots 11 that emerge in the lower openings of said sleeves. The latter are equipped, at each of their parts delimiting said lower openings, with a pair of complementary self-gripping strips 12 of the Velcro® type, one strip of said pair being fastened on one of the parts of the sleeve delimiting said slot 11 and the complementary strip being fastened on the other of said parts and protruding therefrom so as to be able to cover the first strip and engage with it.

The lower openings of the sleeves can thus be adapted to the different arm sizes of the patients.

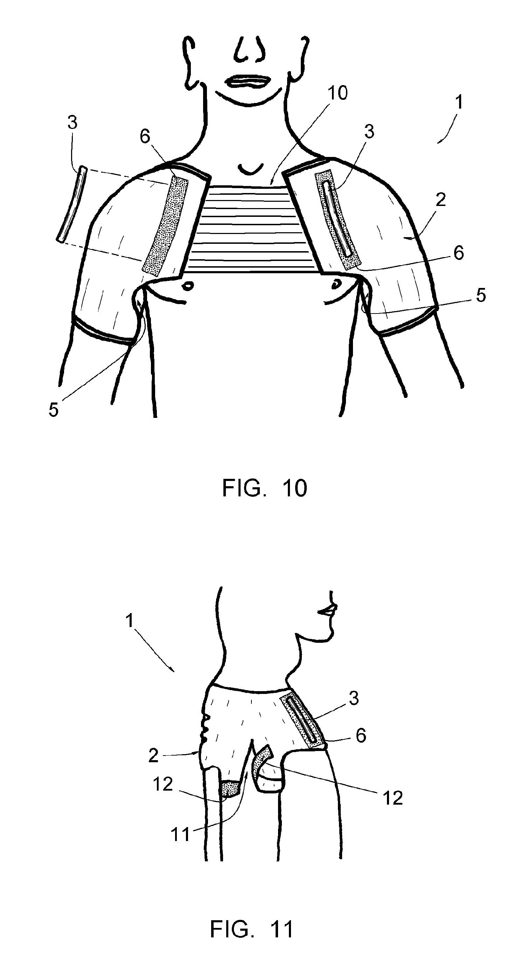

Figures 10 and 11 show still another possible embodiment of the apparatus 1, in which the clothing 2 comprises an elastic forward median part 10 formed by a single elastic strip, and adjustable sleeves comprising the slots 11 on their lateral sides, as well as pairs of complementary self-gripping strips 12 as mentioned above.

The invention has been described in reference to embodiments provided as examples. Of course the invention is not limited to these embodiments, but on the contrary encompasses all other embodiments and alternatives covered by the appended claims.

## Claims

1. An apparatus (1) for treating clavicle fractures, comprising:
- a clothing (2) able to surround the patient's shoulders and the upper part of the patient's arms and thorax in an adjusted manner, comprising cutouts (5) in its areas intended to extend at the patient's axillae;
- elongate anterior reinforcements (3), connected to the clothing (2);
- a tightening means (4) situated at the dorsal part of the clothing (2), able to perform tightening at the patient's shoulder blades;
**characterized in that**:
- said elongate anterior reinforcements (3) comprise two reinforcements intended to extend at the patient's delto-pectoral grooves when the apparatus (1) is worn, and
- said tightening means (4) is adapted to perform essentially transverse tightening, over at least the entire height of the shoulder blades.

2. The apparatus (1) as claimed in claim 1, wherein the clothing (2) comprises an anterior middle part that is elastic over its entire height, said anterior middle part being able to be stretched in the transverse direction of the clothing (2), i.e. in a substantially horizontal direction when said clothing (2) is worn.

3. The apparatus (1) as claimed in claim 1 or in claim 2, wherein the clothing (2) comprises sleeves having lateral slots that emerge in the lower openings of said sleeves, and a means for reclosing said lower openings.

4. The apparatus (1) as claimed in claim 3, wherein said reclosing means of at least one lower openings can in particular be formed by a pair of complementary self-gripping bands of the Velcro® type, one band of said pair being fastened on one of the parts of the sleeve delimiting said slot and the complementary band being fastened on the other of said parts and protruding therefrom so as to be able to cover the first band and to engage with it.

5. The apparatus (1) as claimed in claims 1 to 4, wherein the clothing (2) comprises forward openings favoring aeration.

6. The apparatus (1) as claimed in claims 1 to 5, wherein the clothing (2) comprises a bias, possibly padded, at all of its edges.

7. The apparatus (1) as claimed in claims 1 to 6, wherein the reinforcements (3) are removable relative to the clothing (2), and wherein the apparatus (1) comprises series of reinforcements (3) of different dimensions.

8. The apparatus (1) as claimed in claim 7, wherein the clothing (2) includes, fastened thereto, one of the types of bands of a pair of complementary self-gripping bands (6), and wherein each reinforcement (3) comprises the other type of these bands (6), fastened to its surface.

9. The apparatus (1) as claimed in claim 7, wherein the removable connecting means of a reinforcement (3) to the clothing (2) is adapted to allow the reinforcement (3) to be connected in several locations on the clothing (2).

10. The apparatus (1) as claimed in claim 8, wherein the first aforementioned type of band (6) has dimensions larger than those of a reinforcement (3).

11. The apparatus (1) as claimed in claims 1 to 10, wherein said essentially transverse tightening means (4) comprises several stepped fasteners situated at the shoulder blades, connected to the strap parts that are fastened to the clothing (2).

12. The apparatus (1) as claimed in claim 11, wherein the fasteners (4) are of type with straps provided with complementary self-gripping strips (8), able to be passed into loops (7) then reclosed on themselves stressed.

## Patentansprüche

1. Vorrichtung (1) zur Behandlung von Clavikulafrakturen, umfassend:
- eine Kleidung (2), die in der Lage ist, die Schultern des Patienten und den oberen Teil der Arme und des Thorax des Patienten in angepasster Weise zu umgeben, mit Aussparungen (5) in seinen Bereichen, die dazu bestimmt sind, sich in die Achselhöhlen des Patienten zu erstrecken;
- mit der Kleidung (2) verbundene längliche vordere Verstärkungen (3),
- eine am dorsalen Teil der Kleidung (2) befindliche Spannvorrichtung (4), die in der Lage ist, ein Spannen an den Schulterblättern des Patienten zu vollführen;
**dadurch gekennzeichnet, dass**:
- die länglichen vorderen Verstärkungen (3) zwei Verstärkungen umfassen, die dazu bestimmt sind, sich am Sulcus deltoideopectoralis des Patienten zu erstrecken, wenn die Vorrichtung (1) getragen wird, und
- die Spannvorrichtung (4) dazu ausgelegt ist, ein im Wesentlichen transversales Spannen über mindestens die gesamte Höhe der Schulterblätter zu vollführen.

2. Vorrichtung (1) nach Anspruch 1, wobei die Kleidung (2) einen vorderen Mittelteil umfasst, der über seine gesamte Höhe elastisch ist, wobei der vordere Mittelteil in der transversalen Richtung der Kleidung (2), d.h. in einer im Wesentlichen horizontalen Richtung, wenn die Kleidung (2) getragen wird, gedehnt werden kann.

3. Vorrichtung (1) nach Anspruch 1 oder Anspruch 2, wobei die Kleidung (2) Ärmel mit seitlichen Schlitzen an den unteren Öffnungen der Ärmel, und eine Vorrichtung zum Wiederverschließen der unteren Öffnungen aufweist.

4. Vorrichtung (1) nach Anspruch 3, wobei die Wiederverschließungsvorrichtung von wenigstens einer unteren Öffnung insbesondere durch ein Paar von komplementären selbsthaftenden Bändern des Velcro®-Typs gebildet werden kann, wobei ein Band des Paares an einem der an den Schlitz angrenzenden Ärmelteile befestigt ist, und wobei das komplementäre Band am anderen der genannten Teile befestigt ist und davon absteht, so dass es in der Lage ist, das erste Band zu bedecken und damit in Eingriff zu gelangen.

5. Vorrichtung (1) nach einem der Ansprüche 1 bis 4, wobei die Kleidung (2) Belüftung begünstigende vordere Öffnungen aufweist.

6. Vorrichtung (1) nach einem der Ansprüche 1 bis 5, wobei die Kleidung (2) ein Schrägband, eventuell gepolstert, an allen ihren Kanten aufweist.

7. Vorrichtung (1) nach einem der Ansprüche 1 bis 6, wobei die Verstärkungen (3) in Bezug zur Kleidung (2) entfernbar sind, und wobei die Vorrichtung (1) eine Reihe von Verstärkungen (3) unterschiedlicher Abmessungen aufweist.

8. Vorrichtung (1) nach Anspruch 7, wobei die Kleidung (2), daran befestigt, einen der Typen von Bändern eines Paars von komplementären selbsthaftenden Bändern (6) aufweist, und wobei jede Verstärkung (3), an seiner Oberfläche befestigt, den anderen Typ dieser Bänder (6) aufweist.

9. Vorrichtung (1) nach Anspruch 7, wobei die lösbare Verbindung einer Verstärkung (3) an der Kleidung (2) dazu ausgelegt ist, es der Verstärkung (3) zu gestatten, an mehreren Stellen mit der Kleidung (2) verbunden zu werden.

10. Vorrichtung (1) nach Anspruch 8, wobei der erste vorgenannte Typ von Band (6) Abmessungen hat, die größer als die der Verstärkung (3) sind.

11. Vorrichtung (1) nach einem der Ansprüche 1 bis 10, wobei die im Wesentlichen transversale Spannvorrichtung (4) mehrere an den Schulterblättern befindliche, gestufte Befestigungselemente aufweist, die mit den Bandteilen verbunden sind, welche an der Kleidung (2) befestigt sind.

12. Vorrichtung (1) nach Anspruch 11, wobei die Befestigungselemente (4) vom Typ mit Bändern mit komplementären selbsthaftenden Streifen (8) sind, die in der Lage sind, in Ringe (7) geführt und dann gespannt auf sich selbst wiederverschlossen zu werden.

## Revendications

1. Un appareillage (1) pour le traitement des fractures de clavicule, comprenant :
- un vêtement (2) apte à envelopper de manière ajustée les épaules et la partie supérieure des bras et du thorax du patient, comprenant des découpes (5) dans ses zones destinées à s'étendre au niveau des creux axillaires de ce patient ;
- des renforts antérieurs allongés (3), reliés au vêtement (2) ;
- des moyens de serrage (4) situés au niveau de la partie dorsale du vêtement (2), aptes à opérer un serrage au niveau des omoplates du patient ;
**caractérisé en ce que** :
- lesdits renforts antérieurs allongés (3) comprennent deux renforts destinés à s'étendre au niveau des sillons delto-pectoraux du patient lorsque l'appareillage (1) est porté, et
- lesdits moyens de serrage (4) sont tels qu'ils sont aptes à opérer un serrage essentiellement transversal, sur au minimum l'ensemble de la hauteur des omoplates.

2. L'appareillage (1) selon la revendication 1, **caractérisé en ce que** le vêtement (2) comprend une partie médiane antérieure qui est élastique sur l'ensemble de sa hauteur, cette partie médiane antérieure étant apte à être étirée dans le sens transversal du vêtement (2), c'est-à-dire selon une direction sensiblement horizontale lorsque ce vêtement (2) est porté.

3. L'appareillage (1) selon la revendication 1 ou la revendication 2, **caractérisé en ce que** le vêtement (2) comprend des manches présentant des fentes latérales qui débouchent dans les ouvertures inférieures de ces manches, et des moyens de refermeture de ces ouvertures inférieures.

4. L'appareillage (1) selon la revendication 3, **caractérisé en ce que** lesdits moyens de refermeture d'au moins une ouverture inférieure sont formés par une paire de bandes autoagrippantes complémentaires de type Velcro®" une bande de cette paire étant fixée sur l'une des parties de la manche délimitant ladite fente et la bande complémentaire étant fixée sur l'autre de ces parties et dépassant de celle-ci afin de pouvoir venir recouvrir la première bande et venir en prise avec elle.

5. L'appareillage (1) selon l'une des revendications 1 à 4, **caractérisé en ce que** le vêtement (2) comprend des ouvertures antérieures favorisant l'aération.

6. Appareillage (1) selon l'une des revendications 1 à 5, **caractérisé en ce que** le vêtement (2) comprend un biais, éventuellement rembourré, au niveau de toutes ses bordures.

7. Appareillage (1) selon l'une des revendications 1 à 6, **caractérisé en ce que** les renforts (3) sont amovibles par rapport au vêtement (2), et **en ce que** l'appareillage (1) comprend des séries de renforts (3) de différentes dimensions.

8. Appareillage (1) selon la revendication 7, **caractérisé en ce que** le vêtement (2) comporte, fixé à lui, l'un des types de bandes d'une paire de bandes auto-agrippantes complémentaires (6), et **en ce que** chaque renfort (3) comprend l'autre type de ces bandes (6), fixé à l'une de ses faces.

9. Appareillage (1) selon la revendication 7, **caractérisé en ce que** les moyens de liaison d'un renfort (3) au vêtement (2) sont tels qu'ils permettent la liaison amovible du renfort (3) en plusieurs emplacements du vêtement (2).

10. Appareillage (1) selon la revendication 8, **caractérisé en ce que** ledit premier type de bande (6) présente des dimensions supérieures à celles d'un renfort (3).

11. Appareillage (1) selon l'une des revendications 1 à 10, **caractérisé en ce que** lesdits moyens de serrage essentiellement transversal comprennent plusieurs attaches (4) étagées situées au niveau des omoplates, reliées à des parties de sangles qui sont fixées au vêtement (2).

12. Appareillage (1) selon la revendication 11, **caractérisé en ce que** les attaches (4) sont du type à sangles pourvues de bandes auto-agrippantes complémentaires (8), aptes à être passées dans des boucles (7) puis à être refermées sur elles-mêmes en tension.
